# FASCICULE DE BREVET EUROPEEN

(11) **EP 1 101 445 B1**
(45) Date de publication et mention de la délivrance du brevet: **03.12.2008**
(21) Numéro de dépôt: 00403148.0
(22) Date de dépôt: 13.11.2000
(51) Int. Cl.: A61B 17/08

(54) **Dispositif permettant la fermeture des bords ouverts d'une plaie sans suture**
Vorrichtung zum Zusammenfügen von Wundränden ohne Naht
Device for joining the lips of a wound without suture

(30) Priorité: 22.11.1999 FR 9914632
(43) Date de publication de la demande: 23.05.2001
(73) Titulaire: Detour, Didier, 28300 Saint Prest (FR)
(72) Inventeur: Detour, Didier, 28300 Saint Prest (FR)
(74) Mandataire: Portal, Gérard

(56) Documents cités:
- US-A- 2 371 978
- US-A- 2 722 220
- US-A- 3 487 836
- US-A- 4 430 998
- US-A- 5 534 010

## Description

La présente invention concerne un dispositif permettant la fermeture non traumatique, sans suture, des bords ouverts d'une plaie de la peau d'un mammifère, tel qu'un animal ou un être humain, et le maintien des bords ouverts de la plaie l'un contre l'autre ainsi que les tissus sous-cutanés en favorisant ainsi le processus de guérison avec aération au niveau de la plaie. L'invention permet de réaliser une fermeture étanche de la peau, sans traverser celle-ci tout en permettant une aération au niveau de la plaie, c'est-à-dire que le dispositif selon l'invention ne couvre pas la plaie.

Divers dispositifs sont bien connus à l'homme de l'art pour le traitement d'une plaie traumatique ou chirurgicale qui présentent chacun des avantages et des inconvénients, soit en terme de résultat esthétique à long terme soit en temps de réalisation ou en confort, c'est-à-dire en terme de douleur ressentie par l'individu pendant la période de cicatrisation ou au moment de la pose ou de l'enlèvement du dispositif retenu.

Le dispositif le plus simple est un fil qui permet de coudre des bords ouverts de la plaie pour assurer une cohésion des couches superficielles. Il offre cependant moins cette propriété de cohésion au niveau des couches profondes de la peau. Il présente l'inconvénient en traversant la peau d'engendrer douleur et cicatrice. La technique, dite de Blair-Donati, résout le problème du défaut de rapprochement des couches profondes de la peau en créant ainsi plus de douleur et de cicatrice ultérieure de sorte que le délai de guérison de la plaie s'en trouve augmenter.

Il est aussi connu une technique du surjet intradermique offrant une meilleure cicatrisation superficielle mais qui ne maintient pas suffisamment les tissus sous-cutanés, d'où un élargissement ultérieur de la cicatrice. Le défaut de cette technique est de nécessiter à une procédure plus longue et très difficile à réaliser.

On connaît aussi le système d'agrafe dont divers modèles existent présentant l'avantage d'une grande rapidité d'installation en gardant le défaut d'un corps étranger au sein des différentes structures de la peau, ainsi que de traverser celle-ci et donc de générer des cicatrices ultérieures.

Il est également connu l'emploi de bande adhésive type "sparadrap" qui présente au niveau de la plaie une zone sans adhésif, pour maintenir les deux bords de la plaie en position rapprochée sans toucher la plaie. Un dispositif similaire est décrit dans le brevet US 5,665,108, mais le défaut majeur des solutions de type bande adhésive est que l'aération est très limitée au niveau de la plaie, ce qui engendre des processus de fermentation qui empêchent la guérison.

Il a été proposé dans le brevet US 4,815,468 un dispositif de fermeture sans suture pour tirer et fermer les bords ouverts d'une plaie tout en les maintenant fermement en place, basé sur un système ressort en V inversé, les bords du V présentant des zones planes coopérant avec une bande adhésive. Ce système en V inversé permet par rapprochement des bords du V de ramener les bords ouverts de la plaie en contact l'un contre l'autre. Cependant, ce système présente l'inconvénient majeur d'empêcher toute aération au niveau de la plaie.

Le document US 5,534,010 décrit un dispositif de fermeture sans suture. Lorsque l'on exerce une traction, des éléments en polyamide exercent une poussée sur la plaie. De par la structure du dispositif, l'élément pivote lors de cette traction. Cependant, le moyen de liaison est disposé à l'une des extrémités et ne définit pas le point de pivotement de l'élément. Cet effet de pivotement n'est en outre pas un résultat recherché dans ce document.

La présente invention a pour but principal de résoudre le nouveau problème technique consistant en la fourniture d'un dispositif permettant la fermeture non traumatique des bords ouverts d'une plaie de la peau, sans suture, tout en assurant des forces de cohésion des tissus sous-cutanés pendant une période suffisamment longue pour obtenir leur soudure, en permettant d'obtenir à long terme une cicatrice de meilleure qualité.

La présente invention a encore pour but de résoudre le nouveau problème technique énoncé ci-dessus, selon une solution qui conserve la possibilité de soin, d'asepsie, de contrôle visuel et d'aération au niveau de la plaie, pendant la phase de cicatrisation.

La présente invention a encore pour but principal de résoudre les nouveaux problèmes techniques énoncés ci-dessus, selon une solution qui présente l'avantage de fonctionner sans corps étranger au sein des téguments en limitant ainsi le risque infectieux.

La présente invention a encore pour but principal de résoudre les nouveaux problèmes techniques énoncés ci-dessus, selon une solution qui soit particulièrement simple, qui ne nécessite qu'un minimum de composants du dispositif, particulièrement aisée à mettre en oeuvre par tout usager, sans formation particulière en permettant ainsi un usage de soin accessible dans chaque foyer, et également à un prix réduit.

L'ensemble des problèmes techniques énoncés ci-dessus est résolu par la présente invention de manière simultanée, pour la première fois d'une manière particulièrement simple et à un faible coût.

Ainsi, selon un premier aspect, la présente invention fournit un dispositif permettant la fermeture des bords ouverts d'une plaie de la peau d'un mammifère, tel qu'un animal ou un être humain, sans suture, avec maintien des bords de la plaie l'un contre l'autre en favorisant le processus de guérison, caractérisé en ce qu'il comprend :
a) au moins un premier élément allongé comprenant une première et une deuxième extrémité, ladite première extrémité étant destinée à venir prendre appui sur la peau au voisinage d'un premier bord ouvert de la plaie, ladite deuxième extrémité étant reliée à des premiers moyens de traction qui sont prévus, à l'opposé de la plaie pour exercer une traction de basculement de la première extrémité en direction de la plaie ;
b) au moins un deuxième élément allongé comprenant une première et une deuxième extrémité, ladite première extrémité étant destinée à venir prendre appui sur la peau au voisinage du deuxième bord ouvert de la plaie, et la deuxième extrémité étant reliée à des seconds moyens de traction qui sont prévus, à l'opposé de la plaie, pour exercer une traction de basculement de la première extrémité en direction de la plaie ; et
c) au moins un moyen de liaison est prévu entre lesdits premier et deuxième éléments pour solidariser les deux éléments entre eux, ledit moyen de liaison étant disposé entre lesdites première et deuxième extrémités de chaque premier et deuxième élément afin de définir un point de pivotement de chaque premier et deuxième élément.

Avantageusement, le moyen de liaison est réalisé en un matériau sensiblement non extensible.

Selon un mode de réalisation avantageux de l'invention, le dispositif précité est caractérisé en ce que la première extrémité de chaque élément présente une surface suffisante pour exercer une compression ponctuelle minimale sur la peau.

Selon un autre mode de réalisation avantageux de l'invention, les moyens de traction précités comprennent des moyens adhésifs reliés à la deuxième extrémité, destinés à venir adhérer à une zone saine de la peau à une distance prédéterminée de la plaie.

Selon un autre mode de réalisation avantageux du dispositif selon l'invention, le premier élément précité est sensiblement identique au deuxième élément précité, en facilitant ainsi la procédure de fabrication.

Selon un autre mode de réalisation avantageux de l'invention, le premier élément et le deuxième élément précités présentent une partie inférieure et une partie supérieure, le moyen de liaison précité étant disposé sensiblement à la jonction entre la partie inférieure et la partie supérieure.

Selon un mode de réalisation particulier, la partie supérieure est progressivement effilée jusqu'à aboutir au moyen de traction précité.

Selon un mode de réalisation actuellement préféré, le moyen de liaison précité comprend au moins un fil, de préférence au moins deux fils, de longueur prédéterminée pour permettre à chaque premier et deuxième élément d'être disposés de chaque côté de la plaie. Avantageusement, ce fil est réalisé en un matériau sensiblement inextensible ayant une bonne résistance mécanique, tel qu'un fil de Nylon ou similaire.

Selon un autre mode de réalisation avantageux de l'invention, la première extrémité de chaque premier et deuxième élément précités est réalisé en un matériau permettant d'assurer une adhérence et un respect de la peau près du bord de la plaie en regroupant l'énergie de rapprochement transmise à celle-ci, telle que par exemple une matière hydrocolloïde ou une matière assurant la même fonction.

Avantageusement, lorsque la plaie ouverte présente une longueur supérieure importante, la fermeture de la plaie est obtenue avec plusieurs dispositifs disposés côte à côte.

Afin de diminuer le risque d'arrachement, il pourra être prévu une couche supérieure de protection aérée, par exemple de type "non tissée".

On notera que grâce à l'invention, qui comprend un moyen de liaison tel qu'un fil, il est aisé d'assurer la liaison mécanique entre le premier élément et le deuxième élément du dispositif selon l'invention disposés de part et d'autre de la plaie, tout en assurant un passage de l'air et éventuellement des agents ou produits de soin éventuellement nécessaires en cours de processus de guérison, ce qui distingue l'invention fondamentalement des dispositifs antérieurement connus.

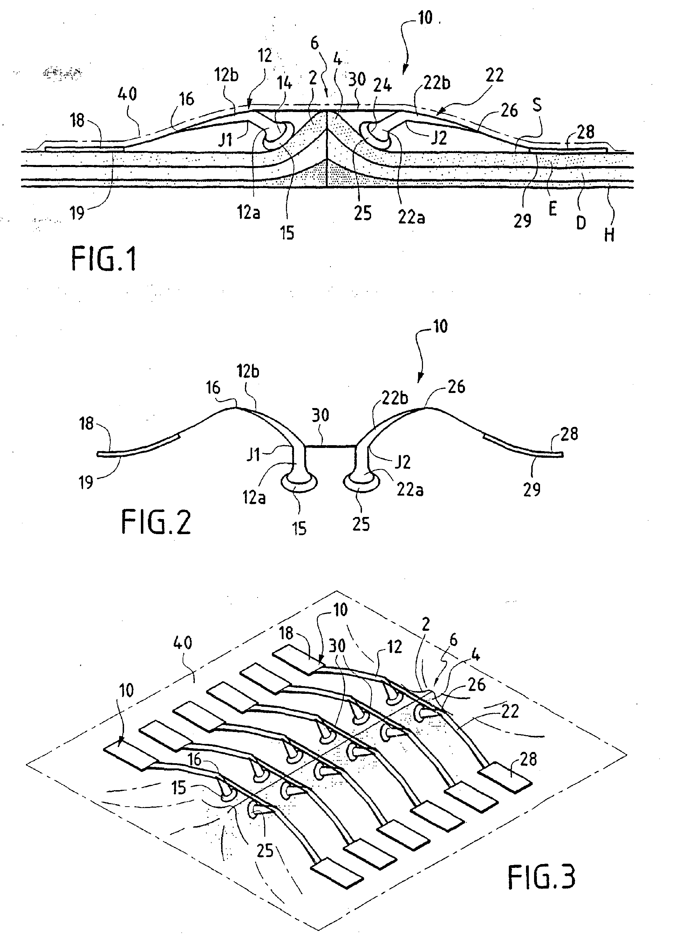

En référence à la figure 1, on a représenté en vue en coupe perspective illustrant un mode de réalisation actuellement préféré d'un dispositif sans suture selon la présente invention en position opératoire avec les bords ouverts d'une blessure en position rapprochée l'un contre l'autre avec rapprochement des tissus sous-jacents comprenant le derme et l'hypoderme;
- la figure 2 représente le dispositif selon l'invention de la figure 1 avant son usage ; et
- la figure 3 représente une vue en perspective de plusieurs dispositifs selon l'invention disposés en position opératoire pour refermer une plaie d'une certaine longueur, permettant d'observer la fermeture sans suture, non traumatique obtenue selon l'invention, les dispositifs étant recouverts d'un dispositif de protection aéré non tissé.

En référence aux figures 1 à 3, un dispositif selon l'invention est représenté par le numéro de référence général 10. Ce dispositif 10 permet la fermeture sans suture, non traumatique, des bords 2, 4 d'une plaie 6 réalisée dans l'épiderme E et traversant également le derme D et l'hypoderme H. La surface S de l'épiderme étant communément appelée la peau.

Le dispositif 10 selon l'invention comprend :
a) au moins un premier élément 12 allongé comprenant une première extrémité 14 et une deuxième extrémité 16. La première extrémité 14 est destinée à venir prendre appui sur la peau S au voisinage du premier bord 2 ouvert de la plaie 6, et la deuxième extrémité 16 étant reliée à des premiers moyens de traction 18 qui sont situés à l'opposé de la plaie 6, comme bien visible à la figure 1, pour exercer une traction de basculement de la première extrémité 14 en direction de la plaie 6 comme cela est bien compréhensible pour un homme de l'art à partir de la considération des figures 1 et 2 ;
b) au moins un deuxième élément 22 allongé comprenant aussi une première extrémité 24 et une deuxième extrémité 26, ladite première extrémité 24 étant aussi destinée à venir prendre appui sur la peau S au voisinage du deuxième bord ouvert 4 de la plaie 6, ladite deuxième extrémité 26 étant reliée à des seconds moyens de traction 28 qui sont situés à l'opposé de la plaie 6 et prévus pour exercer une traction de basculement de la première extrémité 24 en direction de la plaie 6 comme cela est encore bien compréhensible pour un homme de l'art à partir de la considération des figures 1 et 2 ; et
c) au moins un moyen de liaison 30 est prévu entre lesdits premier élément 12 et deuxième élément 22 solidarisant les deux éléments 12 et 22 entre eux, ledit moyen de liaison 30 étant disposé entre lesdites première 14, 24 et deuxième 16, 26 extrémités de chaque premier élément 12 et deuxième élément 22, afin de définir un point de pivotement de chaque élément 12, 22.

Selon un mode de réalisation avantageux de l'invention, la première extrémité respectivement 14, 24 de chaque élément présente une surface suffisante pour exercer une compression ponctuelle minimum sur la peau S.

Avantageusement, lorsque la longueur de la plaie 6 est importante, la fermeture de plaie 6 est obtenue avec plusieurs dispositifs 10 disposés côte à côte.

Selon un autre mode de réalisation avantageux de l'invention, les moyens de traction 18, 28 comprennent des moyens adhésifs, par exemple une bande adhésive 19, 29, de type classique, reliés à la deuxième extrémité respectivement 16, 26, et destinés à venir adhérer à une zone saine de la peau à une distance prédéterminée de la plaie 6, comme aisément compréhensible à partir de la figure 1.

Selon un autre mode de réalisation avantageux de l'invention, le premier élément 12 et le deuxième élément 22 sont sensiblement identiques ou identiques.

Selon un mode de réalisation particulier de l'invention, le premier élément 12 et le deuxième élément 22 présentent une partie inférieure respectivement 12a, 22a et une partie supérieure respectivement 12b, 22b, le moyen de liaison précité 30 étant disposé sensiblement à la zone de jonction respectivement J1, J2 entre la partie inférieure respectivement 12a, 22a et la partie supérieure respectivement 12b, 22b.

Selon une variante de réalisation avantageuse, la partie supérieure 12b, 22b de chaque élément 12, 22 est progressivement effilée jusqu'à la jonction avec les moyens de traction respectivement 18, 28.

Selon une variante de réalisation actuellement préférée, chaque moyen de liaison 30 comprend au moins un fil, de préférence au moins deux fils, comme représenté en figure 3, de longueur prédéterminée pour permettre à chaque premier élément 12 et deuxième élément 22 d'être disposés de chaque côté de la plaie 6 tout en assurant une liaison mécanique sûre et fiable permettant de réaliser un effet de pression et de basculement de la peau et des tissus sous-cutanés, comprenant l'épiderme, le derme et l'hypoderme, comme représenté à la figure 1 avec maintien bord à bord de ces divers tissus pour fermer complètement la plaie 6, comme représenté à la figure 1.

On comprend que l'invention fournit un dispositif qui comprend un élément, respectivement 12, 22 exerçant un effet de levier permettant par son intermédiaire la transmission d'énergie de cohésion nécessaire à la cicatrisation de la plaie 6. Le dispositif selon l'invention, par l'intermédiaire des premier élément 12 et deuxième élément 22, de transmettre aux couches inférieures de la peau, en particulier le derme et l'hypoderme, une augmentation de pression venant s'ajouter à la traction exercée par les moyens de traction respectivement 18, 28.

On comprend ainsi que l'invention réalise la fermeture des bords ouverts d'une plaie sans suture et d'une façon non traumatique, tout en permettant une aération de la plaie facilitant le processus de guérison.

La première extrémité respectivement 14, 24 de chaque élément respectivement 12, 22 est avantageusement pourvue d'un élément adhésif respectivement 15, 25 permettant de solidariser fermement la première extrémité respectivement 14, 24 avec la peau S au voisinage du premier bord 2 ou deuxième bord 4 de la plaie 6 pour assurer une fixation sûre et fiable de cette extrémité en position.

On comprend que le dispositif selon l'invention est particulièrement simple et aisé à mettre en place.

Ainsi, on fait adhérer tout d'abord l'élément adhésif 15 de la première extrémité 14 du premier élément 12 sur le premier bord 2 de la plaie 6, ensuite, on fait adhérer l'élément adhésif 25 de la première extrémité 24 du deuxième élément 22 sur la peau S au niveau du deuxième bord 4 de la plaie 6.

Ensuite, on exerce soit simultanément, soit successivement, une traction sur le premier moyen de traction 18 et le deuxième moyen de traction 28 jusqu'à ce que les bords ouverts 2, 4 de la plaie 6 viennent en contact complet l'un contre l'autre et ensuite on applique les moyens de traction 18, 28 grâce à leur élément adhésif sur la peau S à une zone adéquate de la peau qui permet de maintenir les bords ouverts 2, 4 de la plaie 6 en contact l'un contre l'autre, comme représenté à la figure 1.

Généralement, la plaie 4 présente une certaine longueur, qui nécessite l'application de plusieurs dispositifs selon l'invention 10, espacés les uns des autres, selon un espacement estimé par le praticien, de manière à assurer une fermeture sans suture, non traumatique, sûre et fiable, comme montré à la figure 3.

Afin de diminuer le risque d'arrachement, il pourra être prévu un dispositif 40 formant une couche supérieure de protection aérée, par exemple de type "non tissé", telle qu'une gaze aérée non tissée, bien connue aux praticiens.

On comprend que l'invention permet, grâce aux moyens de liaison 30, de relier mécaniquement le premier élément 12 et le deuxième élément 22 et, par le positionnement du moyen de liaison sur la zone de jonction J1 ou J2 du premier élément 12 et du deuxième élément 22, d'assurer un effet de pression et de rapprochement non seulement de l'épiderme mais également du derme et de l'hypoderme jusqu'à ce que les bords 2, 4 de la plaie ouverte soient en contact l'un contre l'autre comme représenté à la figure 1.

De préférence, le dispositif selon l'invention est à usage unique.

La réalisation des dimensions et le choix des matériaux pour la mise en oeuvre du dispositif selon l'invention sont également bien apparentes à l'homme de l'art qui n'aura aucune difficulté pour les réaliser.

### EXEMPLE DE REALISATION

Pour cet exemple de réalisation, qui constituera le mode de réalisation préféré, chaque premier élément 12 et deuxième élément 22 est réalisé en matière plastique légère et non allergique, telle que nylon, polyéthylène ou minérale telle que graphite et présente à ses extrémités 12a, 22a une dimension comprise généralement entre 3 et 6 mm. La longueur des fils 30, également réalisés par exemple en nylon, sera généralement aussi comprise entre 3 et 5 mm, le diamètre des fils de nylon n'est pas critique, mais doit être suffisant pour conférer une résistance mécanique sûre et fiable. Ce diamètre pourra être par exemple de 0,1 à 1 mm. Le fil 30 est solidarisé à chaque élément 12, 22 à une distance de l'ordre de 3 à 4 mm, depuis la base constituée par une matière adhésive 15 ou 25. L'élément adhésif 15, 25 sera constitué par un pavé d'hydrocolloïde disponible dans le commerce, et bien connu des praticiens. Les bandes adhésives 19, 29 sont de type classique disponibles dans le commerce, par exemple des bandes adhésives type Steristrip® commercialisées par la société 3M.

La couche de protection 40 sera constituée par une gaze aérée non tissée disponible dans le commerce.

## Revendications

1. Dispositif (10) permettant la fermeture des bords ouverts (2, 4) d'une plaie (6) de la peau (S) d'un mammifère, tel qu'un animal ou un être humain, et maintien des bords (2, 4) l'un contre l'autre en favorisant le processus de guérison, qui comprend :
a) au moins un premier élément (12) allongé comprenant une première extrémité (14) et une deuxième extrémité (16), ladite première extrémité (14) étant destinée à venir prendre appui sur la peau (S) au voisinage du premier bord (2) ouvert de la plaie (6), ladite deuxième extrémité (16) étant reliée à des premiers moyens de traction (18) qui sont prévus à l'opposé de la plaie (6) et prévus pour exercer une traction de basculement de la première extrémité (14) en direction de la plaie (6) ;
b) au moins un deuxième élément (22) allongé comprenant une première extrémité (24) et une deuxième extrémité (26), ladite première extrémité (24) étant destinée à venir prendre appui sur la peau (S) au voisinage du deuxième bord (4) ouvert de la plaie (6), et ladite deuxième extrémité (26) étant reliée à des seconds moyens de traction (28) qui sont prévus" à l'opposé de la plaie (6), pour exercer une traction de basculement de la première extrémité (24) en direction de la plaie (6) ; et
c) au moins un moyen de liaison (30), chaque moyen de liaison étant disposé entre l'un desdits premier éléments (12) et l'un desdits deuxième éléments (22) pour solidariser les deux éléments entre eux,
le dispositif étant **caractérisé en ce que** chaque extrémité de chaque moyen de liaison (30) est disposée entre lesdites première extrémité (14, 24) et deuxième extrémité (16, 26) de chaque premier élément (12) et deuxième élément (22), définissant ainsi un point de pivotement de chaque élément (12, 22).

2. Dispositif selon la revendication 1, **caractérisé en ce que** la première extrémité (14, 24) de chaque élément (12, 22) présente une surface suffisante pour exercer une compression ponctuelle minimum sur la peau (S).

3. Dispositif selon l'une des revendications précédentes, **caractérisé en ce que** les moyens de traction (18, 28) comprennent des moyens adhésifs reliés à la deuxième extrémité (16, 26) de chaque élément (12, 22), destinés à venir adhérer à une zone saine de la peau à une distance prédéterminée de la plaie (6).

4. Dispositif selon l'une des revendications précédentes, **caractérisé en ce que** le premier élément (12) et le deuxième élément (22) sont sensiblement identiques.

5. Dispositif selon l'une des revendications précédentes, **caractérisé en ce que** le premier élément (12) et le deuxième élément (22) présentent une partie inférieure (12a, 22a) et une partie supérieure (12b, 22b), et le moyen de liaison (30) est disposé sensiblement à la jonction (J1, J2) entre la partie inférieure (12a, 22a) et la partie supérieure (12b, 22b).

6. Dispositif selon la revendication 5, **caractérisé en ce que** la partie supérieure (12b, 22b) est progressivement effilée jusqu'à aboutir au moyen de traction (18, 28).

7. Dispositif selon l'une des revendications précédentes, **caractérisé en ce que** le moyen de liaison précité (30) comprend au moins un fil, de préférence au moins deux fils, de longueur prédéterminée pour permettre à chaque premier élément (12) et deuxième élément (22) d'être disposés de chaque côté (2, 4) de la plaie (6).

8. Dispositif selon l'une des revendications précédentes, **caractérisé en ce qu'**il est prévu une couche de protection aérée, par exemple de type "non tissée" (40), afin de diminuer le risque d'arrachement.

9. Dispositif selon l'une des revendications précédentes, **caractérisé en ce que** chaque premier élément (12) et deuxième élément (22) est réalisé en matière plastique légère et non allergique, telle que nylon, polyéthylène ou minérale tel que graphite.

10. Dispositif selon la revendication 9, **caractérisé en ce que** chaque premier élément (12) et deuxième élément (22) présente à ses extrémités (12a, 22a) une dimension comprise généralement entre 3 et 6 mm, chaque fil (30) est solidarisé à chaque élément (12, 22) à une distance de l'ordre de 3 à 4 mm depuis la base constituée par une matière adhésive (15 ou 25), le diamètre du fil étant par exemple de 0,1 à 1 mm.

## Claims

1. Devise (10) for closing the open edges (2, 4) of a wound (6) in the skin (S) of a mammal, such as an animal or a human being, and retaining the edges (2, 4) against each other, for promoting the healing process, which comprises:
a) at least one first elongated element (12) comprising a first end (14) and a second end (16), said first end (14) being intended to come into abutment on the skin (S) near the first open edge (2) of the wound (6), said second end (16) being connected to first traction means (18) which are provided opposite the wound (6) to exert a tilting traction to turn the first end (14) towards the wound (6);
b) at least one second elongated element (22) comprising a first end (24) and a second end (26), said first end (24) being intended to come into abutment on the skin (S) near the second open edge (4) of the wound (6), and said second end (26) being connected to second traction means (28) which are provided, opposite the wound (6), to exert a tilting traction to turn the first end (24) towards the wound (6); and
c) at least one connecting means (30), each connecting means being disposed between one of said first elements (12) and one of said second elements (22) to join the two elements together, the device being **characterized in that** each end of each connecting means (30) is disposed between said first end (14, 24) and second end (16, 26) of each first element (12) and second element (22) thereby defining a pivoting point of each element (12, 22).

2. Device according to claim 1, **characterized in that** the first end (14, 24) of each element (12, 22) presents a sufficient surface to exert a minimum pin-point compression on the skin (S).

3. Device according to any of the preceding claims, **characterized in that** the traction means (18, 28) comprise adhesive means connected to the second end (16, 26) of each element (12, 22), intended to adhere in a healthy zone of the skin at a predetermined distance from the wound (6).

4. Device according to any of the preceding claims, **characterized in that** the first element (12) and the second element (22) are substantially identical.

5. Device according to any of the preceding claims, **characterized in that** the first element (12) and the second element (22) present a lower part (12a, 22a) and an upper part (12b, 22b), and the connecting means (30) is disposed substantially at the join (J1, J2) between the lower part (12a, 22a) and the upper part (12b, 22b).

6. Device according to claim 5, **characterized in that** the upper part (12b, 22b) tapers progressively up to the traction means (18, 28).

7. Device according to any of the preceding claims, **characterized in that** the connecting means (30) comprises at least one yarn, preferably at least two yarns, of predetermined length to allow each first element (12) and second element (22) to be disposed on each side (2, 4) of the wound (6).

8. Devise according to any of the preceding claims, **characterized in that** an aerated projective layer (40), for example of "non-woven" type, is provided, in order to reduce the risk of tear.

9. Devise according to any of the preceding claims, **characterized in that** each first element (12) and second element (22) is made of light, non-allergic plastics material such as nylon, polyethylene, or mineral such as graphite.

10. Devise according to claim 9, **characterized in that** each first element (12) and second element (22) present at its ends (12a, 22a) a dimension generally included between 3 and 6 mm, each yarn (30) is connected at each element (12, 22) at a distance of the order of 3 to 4 mm from the base constituted by an adhesive material (15 or 25), the diameter of the yarn being for example from 0.1 to mm.

## Patentansprüche

1. Vorrichtung (10), die das Verschließen der offenen Ränder (2, 4) einer Wunde (6) der Haut (S) eines Säugers, wie eines Tiers oder eines Menschen, sowie das Aneinanderhalten der Ränder (2, 4) ermöglicht und dabei den Heilungsprozeß begünstigt und die folgendes umfaßt:
a) wenigstens ein erstes längliches Element (12), das ein erstes Ende (14) und ein zweites Ende (16) aufweist, wobei das erste Ende (14) dazu bestimmt ist, an der Haut (S) in der Nähe des ersten offenen Randes (2) der Wunde (6) zur Anlage zu kommen, wobei das zweite Ende (16) mit ersten Zugmitteln (18) verbunden ist, die gegenüber der Wunde (6) vorgesehen und dazu vorgesehen sind, einen Zug zum Kippen des ersten Endes (14) in Richtung der Wunde (6) auszuüben,
b) wenigstens ein zweites längliches Element (22), das ein erstes Ende (24) und ein zweites Ende (26) aufweist, wobei das erste Ende (24) dazu bestimmt ist, an der Haut (S) in der Nähe des zweiten offenen Randes (4) der Wunde (6) zur Anlage zu kommen, und das zweite Ende (26) mit zweiten Zugmitteln (28) verbunden ist, die gegenüber der Wunde (6) dazu vorgesehen sind, einen Zug zum Kippen des ersten Endes (24) in Richtung der Wunde (6) auszuüben, und
c) wenigstens ein Verbindungsmittel (30), wobei jedes Verbindungsmittel zwischen einem der ersten Elemente (12) und einem der zweiten Elemente (22) angeordnet ist, um die beiden Elemente fest miteinander zu verbinden,
wobei die Vorrichtung **dadurch gekennzeichnet ist, daß** jedes Ende eines jeden Verbindungsmittels (30) zwischen dem ersten Ende (14, 24) und dem zweiten Ende (16, 26) jedes ersten Elements (12) und zweiten Elements (22) angeordnet ist, wodurch ein Punkt zum Verschwenken jedes Elements (12, 22) definiert wird.

2. Vorrichtung nach Anspruch 1, **dadurch gekennzeichnet, daß** das erste Ende (14, 24) jedes Elements (12, 22) eine Fläche aufweist, die ausreichend ist, um einen minimalen punktuellen Druck auf die Haut (S) auszuüben.

3. Vorrichtung nach einem der vorhergehenden Ansprüche, **dadurch gekennzeichnet, daß** die Zugmittel (18, 28) Haftmittel umfassen, die mit dem zweiten Ende (16, 26) jedes Elements (12, 22) verbunden und dazu bestimmt sind, an einem gesunden Bereich der Haut in einem vorbestimmten Abstand von der Wunde (6) zu haften.

4. Vorrichtung nach einem der vorhergehenden Ansprüche, **dadurch gekennzeichnet, daß** das erste Element (12) und das zweite Element (22) im wesentlichen identisch sind.

5. Vorrichtung nach einem der vorhergehenden Ansprüche, **dadurch gekennzeichnet, daß** das erste Element (12) und das zweite Element (22) einen unteren Teil (12a, 22a) sowie einen oberen Teil (12b, 22b) aufweisen und daß das Verbindungsmittel (30) im wesentlichen an der Verbindungsstelle (J1, J2) zwischen dem unteren Teil (12a, 22a) und dem oberen Teil (12b, 22b) angeordnet ist.

6. Vorrichtung nach Anspruch 5, **dadurch gekennzeichnet, daß** sich der obere Teil (12b, 22b) bis zum Erreichen des Zugmittels (18, 28) nach und nach verjüngt.

7. Vorrichtung nach einem der vorhergehenden Ansprüche, **dadurch gekennzeichnet, daß** das vorgenannte Verbindungsmittel (30) wenigstens einen Faden, vorzugsweise wenigstens zwei Fäden vorbestimmter Länge aufweist, um jedem ersten Element (12) und zweiten Element (22) zu ermöglichen, auf jeder Seite (2, 4) der Wunde (6) angeordnet zu werden.

8. Vorrichtung nach einem der vorhergehenden Ansprüche, **dadurch gekennzeichnet, daß** eine belüftete, beispielsweise "vliesartige" Schutzschicht (40) vorgesehen ist, um das Risiko eines Herausreißens zu verringern.

9. Vorrichtung nach einem der vorhergehenden Ansprüche, **dadurch gekennzeichnet, daß** jedes erste Element (12) und Elemente (22) aus leichtem und Kunststoff, wie zum Beispiel Nylon, Polyethylen, oder mineralischem Material, wie zum Beispiel Graphit gefertigt ist.

10. Vorrichtung nach Anspruch 9, **dadurch gekennzeichnet, daß** jedes erste Element (12) und zweite Element (22) an seinen Enden (12a, 22a) eine Abmessung von im allgemeinen 3 bis 6 mm aufweist, wobei jeder Faden (30) mit jedem Element (12, 22) in einem Abstand in der Größenordnung von 3 bis 4 mm von der von einem Haftmaterial (15 oder 25) gebildeten Basis aus fest verbunden ist, wobei der Durchmesser des Fadens beispielsweise 0,1 bis 1 mm beträgt.
